Europäisches Patentamt

European Patent Office

Office européen des brevets

(19)

(11) **EP 0 586 385 B2**

(12) **NEUE EUROPÄISCHE PATENTSCHRIFT**

(45) Veröffentlichungstag und Bekanntmachung des Hinweises auf die Entscheidung über den Einspruch:
**30.03.2005 Patentblatt 2005/13**

(45) Hinweis auf die Patenterteilung:
**06.11.1996 Patentblatt 1996/45**

(21) Anmeldenummer: **92905421.1**

(22) Anmeldetag: **28.02.1992**

(51) Int Cl.$^7$: **B01D 15/08**, C07B 57/00

(86) Internationale Anmeldenummer:
**PCT/EP1992/000439**

(87) Internationale Veröffentlichungsnummer:
**WO 1992/016274 (01.10.1992 Gazette 1992/25)**

(54) **VERFAHREN ZUR TRENNUNG VON ENANTIOMEREN AN CHIRALEN TRENNPHASEN MIT HILFE EINES KONTINUIERLICHEN GEGENSTROM-CHROMATOGRAPHIEVERFAHRENS**

ENANTIOMER SEPARATING PROCESS ON CHIRAL SEPARATION PHASES BY MEANS OF A CONTINUOUS COUNTER-CURRENT CHROMATOGRAPHY PROCESS

PROCEDE DE SEPARATION D'ENANTIOMERES DANS DES PHASES DE SEPARATION CHIRALES PAR UN PROCEDE DE CHROMATOGRAPHIE EN CONTINU A CONTRE-COURANT

(84) Benannte Vertragsstaaten:
**AT BE CH DE DK ES FR GB GR IT LI LU NL SE**

(30) Priorität: **14.03.1991 DE 4108188**

(43) Veröffentlichungstag der Anmeldung:
**16.03.1994 Patentblatt 1994/11**

(73) Patentinhaber: **BAYER AG**
**51368 Leverkusen (DE)**

(72) Erfinder:
• **ARLT, Dieter**
  **D-5000 Köln 80 (DE)**
• **LANGE, Walter**
  **D-5000 Köln 60 (DE)**
• **BÖMER, Bruno**
  **D-5060 Bergisch Gladbach (DE)**
• **GROSSER, Rolf**
  **D-5090 Leverkusen (DE)**
• **PAUL, Hanns-Ingolf**
  **D-5000 Köln 80 (DE)**
• **SCHNABEL, Günter**
  **D-5632 Wermelskirchen (DE)**

(56) Entgegenhaltungen:
EP-A- 0 471 082        DE-A- 2 500 523
DE-A- 2 756 496        FR-A- 2 550 462
US-A- 3 268 605

• "An energy-saving separation scheme", Neuzil et al, CHEMTECH, August 1980, 498-503

EP 0 586 385 B2

## Beschreibung

Die Erfindung geht aus von einem Verfahren zur Trennung eines chiralen Stoffgemisches mit Hilfe eines Gegenstrom-Chromatographieverfahrens, bei dem einer aus einer Vielzahl von hintereinandergeschalteten, mit einem Adsorbens gefüllten Chromatographiesäulen bestehenden Säulenschaltung das zu trennende Stoffgemisch EAB und das Eluens kontinuierlich zugeführt werden und an anderen Stellen der Säulenschaltung ein die Komponente A enthaltender Extraktstrom AE, sowie ein die Komponente B enthaltender Raffinatstrom BE kontinuierlich entnommen werden. Gleichzeitig wird dabei eine Relativbewegung zwischen einer aus dem Stoffgemisch ABE und dem Eluens EO bestehenden flüssigen, mobilen Phase und dem Adsorbens in fester Phase durch sequentielles Öffnen von Flüssigkeitszugabe- und -entnahmestellen entlang der Chromatographiesäulen erzeugt.

In den letzten Jahren gewinnt die Trennung von Wirkstoff-Racematen zunehmend an Bedeutung, weil sich gezeigt hat, daß die Enantiomere eines Wirkstoff-Racemates sich häufig in ihren biologischen Wirkungen und Nebenwirkungen unterscheiden.

Die äußerst schwierige verfahrenstechnische Aufgabe der Gewinnung enantiomerenreiner Produkte wird bereits auf verschiedene Weise gelöst, wobei klassische Verfahren wie enantioselektive Synthese, direkte und indirekte, d.h. über den Umweg von Substitutionsreaktionen o.ä Rektifikationen, Extraktionen mit Hilfsstoffen, fraktionierte Kristallisation und diskontinuierlich betriebene präparative chromatographische Verfahren etc. in der Literatur beschrieben sind. Vor allem den physikalischen Verfahren haftet der Nachteil an, daß sie oft den engen wirtschaftlichen Randbedingungen nicht genügen können.

Besonderes Augenmerk verdienen seit längerem die auf dem Prinzip der selektiven Adsorption basierenden Trennverfahren. Adsorptive Verfahren gewinnen angesichts wachsender Anforderungen an die Produktreinheit und spezifische Wirkungen von Enantiomeren zunehmend an Bedeutung. Dies ist vor allem dann der Fall, wenn die sehr schwierige Trennung von Diastereomeren und Racematen verlangt wird. Den Vorteilen hoher Trennleistung der chromatographischen Verfahren stehen jedoch die Nachteile der diskontinuierlichen Betriebsweise mit oft extremen Verdünnungen gegenüber.

Die adsorptive bzw. chromatographische Trennung eines Stoffgemisches geschieht dadurch, daß diese Komponenten aus einer gasförmigen oder flüssigen Lösung heraus unterschiedliche spezifische Wechselwirkungen mit festen Phasen (den Adsorbentien), die mit geeigneten optisch-aktiven Substanzen belegt sind, ausbilden. Durch Elution mit einem geeigneten gasförmigen oder flüssigen Desorbens (Eluens) können die gewünschten Verbindungen nacheinander selektiv von der stationären Phase gespült und voneinander isoliert werden. Die Selektivität ist hier definiert durch

$$\alpha = \frac{\text{Konzentration A im Zulauf/Konzentration A im Eluat}}{\text{Konzentration B im Zulauf/Konzentration B im Eluat}} \qquad (1).$$

Bei den stationären Phasen kann es sich um Kieselgele, Aktivkohlen, Zeolithe, Ionentauscherharze oder Gele handeln (siehe z.B. US-PS 3 665 046, 3 668 266, 4 319 929 und 4 519 845 für Zeolith, Aktivkohle- und Harz-Adsorbentien). Auch die Verwendung von vernetzten Polymerisaten auf der Basis polymerisierter optisch aktiver Acrylsäureamide oder Methacrylsäureamide als Adsorbentien zur Racemat-Trennung in konventionellen chromatographischen Säulen ist bekannt (siehe DE-A 2 500 523). Als Elutionsmittel sind alle denkbaren Substanzen, die den Trennvorgang in günstiger Weise beeinflussen, geeignet. Die beschriebene diskontinuierliche Prozedur erweist sich jedoch aus technischer Sicht wie eingangs erläutert als unbefriedigend.

Ein kontinuierliches Gegenstrom-Chromatographieverfahren kann beispielsweise durch gravitationsbetriebenen Transport der Adsorbenspartikel gegen den Fluidstrom realisiert werden. Eine andere Verfahrensalternative stützt sich auf das Prinzip des "simulated moving bed" (kurz SMB, US-PS 2 985 589, E. Broughton). Bei diesem Verfahren wird das Adsorbens nicht tatsächlich, sondern nur scheinbar bewegt. Dabei werden die Zugabe- und Entnahmestellen entlang der Chromatographiesäule geführt, die man sich als geschlossenen Ring oder als Folge von Einzelsäulen vorstellen kann. Somit liegt eine Relativbewegung zwischen mobiler fluider und fester Phase vor. Der Flüssigkeits- oder Gasstrom wird im Kreis geführt, so daß das stationäre Konzentrationsprofil ebenfalls umläuft. Es handelt sich also um einen Gegenstromprozeß von Einsatzmischung gegenüber Eluat und Adsorbens. Die bewegte Zugabe und Entnahme der Flüssigkeits- oder Gasströme geschieht jedoch nicht in infinitesimalen, sondern endlichen Schritten, die durch die Höhe der einzelnen Festbettstufen bestimmt sind. Insgesamt teilt sich die Festbettlänge in vier Sektionen: Der Rektifikationszone, der Verstärkerzone, der Abtriebszone und der Pufferzone. Die Schaltung der Ventile für die Zu- und Ablaufströme kann über einzelne separate Ventile oder eine zentrale Verteilereinrichtung (Rotationsscheiben-Ventil US-PS 3 040 777, 3 422 848) erfolgen. Anwendungen sind aus dem Bereich der Olefin-, Aromaten- und Zuckertrennungen bekannt und beschrieben (US-PS 3 205 166, 3 291 726, 3 416 961, 3 510 423 sowie 3 707 550 und 4 157 267 etc.). Insbesondere wird in US-A-3 268 605 ein übergeordnetes Regelsystem für ein kontinuierliches Gegenstrom-Chromatographieverfahren auf der Basis einer mit sequentiellen Anschlußleitungen versehenen, chromatographischen Säule in Verbindung mit einem Multikanaldrehventil beschrieben. Der chromatographischen Säule wird das zu trennende Produkt (Racemat) und ein Desorbens (Eluens) kontinuierlich zugeführt, während ein Sorbatstrom (Extrakt) und ein Raffinatstrom kontinierlich abgeführt werden. Das Regelsystem ist so ausgeführt, daß drei der vier Produktströme Mengenstrom-geregelt sind, während der vierte Produktstrom so gedrosselt wird, daß ein konstanter Gegendruck in der Kontaktzone aufrechterhalten wird. Insbesondere werden der zugeführte Produkt- und Eluensstrom auf einen vorgegebenen Wert eingestellt und entweder eine oder beide Ausgangsproduktströme für Regelzwekke gedrosselt. Die zugrundeliegenden Regelalgorithmen werden ausführlich beschrieben. Als stationäre Phase werden in der chromatographischen Trennsäule sogenannte Molekularsiebe aus dehydrierten Metall-Aluminiumsilikaten verwendet.

Bisher ist die Trennung von Enantiomeren mit Hilfe kontinuierlich betriebener Gegenstrom-Chromatographieverfahren nicht gelungen. Das erfindungsgemäße Verfahren geht aus von der Trennung eines Stoffgemisches durch ein kontinuierlich betriebenes Gegenstrom-Chromatographieverfahren, bei dem eine Relativbewegung zwischen einer aus dem Stoffgemisch und einer flüssigen mobilen Phase und einem in einer Vielzahl von hintereinander geschalteten Chromatographiesäulen befindlichen Adsorbens in fester Phase durch sequentielles Öffnen von Flüssigkeitszugabeund Entnahmestellen entlang der Säulen erzeugt wird.

Der Kern des erfindungsgemäßen Verfahrens besteht darin, daß der mit einem chiralen Polymer als Adsorbens in Form einer Festbettschüttung gefüllten Säulenschaltung das enantiomaere Stoffgemisch ABE (A = 1.Komponenten, B = 2.Komponente, E = Eluens) zugeführt wird, wobei die Mengenströme EO, ABE, AE und BE (EO = Eluensstrom, AE = 1.Komponente + Eluens [= Extrakt], BE = 2.Komponente + Eluens [= Raffinat] konstant geregelt werden, während bei einem voreingestellten Systemdruck in der diesem Teilstrom führenden Zuleitung oder so nachgeregelt wird, daß der Systemdruck konstant bleibt und wobei der in Strömungsrichtung nach dem Mengenstrom-Stellglied herrschende Systemdruck in der Eluens-Zuleitung als Regelgröße verwendet wird. Bei dem vierten Teilstrom wird also der Systemdruck als Regelgröße benutzt. Dies bedeutet, daß sich der vierte Mengenstrom unter Einhaltung eines konstanten Systemdrucks frei einstellen kann. Die sorgfältige Einhaltung der Mengenströme der vier dem Prozeß zugeleiteten und abgeführten Ströme schafft die Voraussetzungen, daß auch druckempfindliche (gegenüber Druckschwankungen!) chirale Polymere mit gleichbleibend gutem Wirkungsgrad unter konstanten Betriebsbedingungen als Adsorbens in Form einer Festbettschüttung zur Trennung eines racemischen Stoffgemisches eingesetzt werden können.

Gemäß einer Weiterentwicklung des Verfahrens werden im stationären Betriebszustand die Konzentrationsverläufe der beiden enantiomeren Komponenten A, B längs der Säulenschaltung gemessen und das zu trennende Stoffgemisch ABE an der dem Schnittpunkt der beiden Konzentrationsprofile zunächst benachbarten Flüssigkeitsaufgabestelle zwischen den Säulen zugeführt.

Als chirale Adsorbentien für die Enantiomerentrennung kommen vorzugsweise zum Einsatz:: Vernetzte Perlpolymerisate auf der Basis von optisch aktiven Acryl-, Methacryl- und $\alpha$-Fluoracrylamiden; Polymere aus Triphenylmethylmethacrylaten und davon abgeleiteten Derivaten; Celluloseester und -carbamate, wie z.B. Cellulosetriacetat und Cellulosetriphenylcarbamat; ferner anorganische Träger wie z.B. Kieselgele, die mit den zuvor genannten Polymeren belegt sind. Die zuvor erwähnten Perlpolymerisate sollten vorzugsweise eine Korngröße zwischen 10 µm und 150 µm und einen Quellungsgrad zwischen 3 ml)/g und 9 ml/g aufweisen.

Mit dem erfindungsgemäßen Verfahren können zum ersten Male enantiomere Stoffgemische unter sehr stabilen und selbstregulierenden Prozeßbedingungen getrennt werden. Kleine bis mittlere Störungen durch Schwankungen der Temperatur und der Konzentration des Einsatzproduktes werden dabei ohne steuernde Eingriffe ausgeglichen. Die besonderen Vorzüge des Verfahrens liegen in der gleichmäßigen Betriebsweise mit konstanten Produktqualitäten, höheren Konzentrationen, größeren Raum-/Zeit-Ausbeuten bei hohen Standzeiten sowie dem geringeren Bedarf an Trennmaterial (Adsorbens). Damit wurde ein technischer Weg für die kontinuierliche Produktion von enantiomerenreinen Verbindungen eröffnet.

Es zeigen:

Fig. 1 ein strömungstechnisches Ersatzschaltbild zur Erläuterung der prinzipiellen Prozeßführung,

Fig. 2 das Prinzip der Säulenschaltung zur Realisierung des chromatographischen Gegenstromprozesses,

Fig. 3 ein Fließbild für die verfahrenstechnische Realisierung und

Fig. 4 eine Momentaufnahme des Konzentrationsprofils für die beiden zu trennenden Enantiomeren-Komponenten A, B während des Betriebs der Anlage.

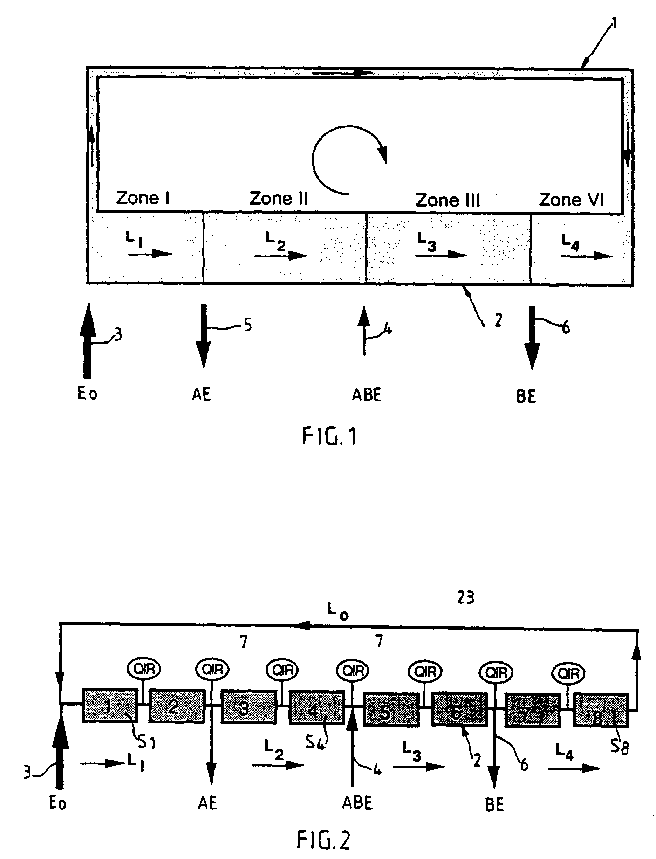

Aus Gründen der Anschaulichkeit bei der Erläuterung der prinzipiellen Prozeßbedingungen wird gemäß Fig. 1 unterstellt, daß das Adsorbens (stationäre Phase) im Kreislauf 1 durch die Säulenschaltung 2 geführt wird. Längs der Säulenschaltung 2 sind die Zuläufe 3 und 4 für das Eluens EO und die mit dem Eluens verdünnte Mischung ABE der zu trennenden Enantiomeren-Komponenten A, B sowie die Abläufe 5 und 6 für den Extrakt AE (abgetrennte Komponente A) und das Raffinat BE (abgetrennte Komponente B) angeordnet. In Wirklichkeit verbleibt jedoch das Adsorbens als ortsfeste Festbettschüttung in der Säulenschaltung und die Gegenstrombewegung wird dadurch simuliert, daß die Zulauf- und Entnahmestellen entlang der Säulenschaltung 2 wandern, d.h. sukzessive ein- bzw. ausgeschaltet werden. Dadurch wird die stärker adsorbierte Komponente A gegenüber dem bewegten Zulauf 4 zurückgehalten, während die andere Komponente B dem Zulauf vorauseilt. Einem mit der bewegten Zulaufstelle 4 wandernden Beobachter scheint somit der Feststoff entgegenzuströmen, während er vom Eluens EO überholt wird. Gemäß Fig. 1 kann die Säulenschaltung 2 in vier Zonen unterteilt werden. Die Zone I erstreckt sich vom Eluenszulauf 3 bis zum Extraktablauf 5, die Zone II vom Extraktablauf 5 bis zum Mischungszulauf 4, die Zone III vom Zulauf 4 bis zum Raffinatablauf 6 und die Zone IV vom Raffinatablauf 6 bis zum Ende der Säule. In der Zone I wird die Komponente A nach rechts in Richtung Extraldablauf 5 und in der Zone II nach links in Richtung Extraktablauf 5 gefördert. Dagegen wird in der Zone III die Komponente B nach rechts in Richtung Raffinatablauf 6 und in der Zone IV nach links in Richtung Raffinatablauf 6 gefördert. Die zu den einzelnen Zonen gehörenden Flüssigkeitsteilströme sind mit $L_1$, $L_2$, $L_3$ und $L_4$ bezeichnet.

Gemäß Fig. 2 wird die Säulenschaltung 2 durch acht hintereinander geschaltete Trennsäulen $S_1$ bis $S_8$ realisiert. Die Zone I besteht also hier aus den Säulen $S_1$ und $S_2$, die Zone II aus den Säulen $S_3$ und $S_4$, die Zone III aus den Säulen $S_5$ und $S_6$ und die Zone IV aus den Säulen $S_7$ und $S_8$. Der im Kreislauf geführte Flüssigkeitsstrom wird mit $L_O$ bezeichnet. Zwischen den Säulen sind Meßstellen (QIR) 7 für die Erfassung der Konzentrationen für die Komponenten A und B vorgesehen. Im übrigen gelten die gleichen Bezeichnungen für die Teilströme sowie für die Zuläufe und Abläufe längs der Säulenschaltung wie in Fig. 1.

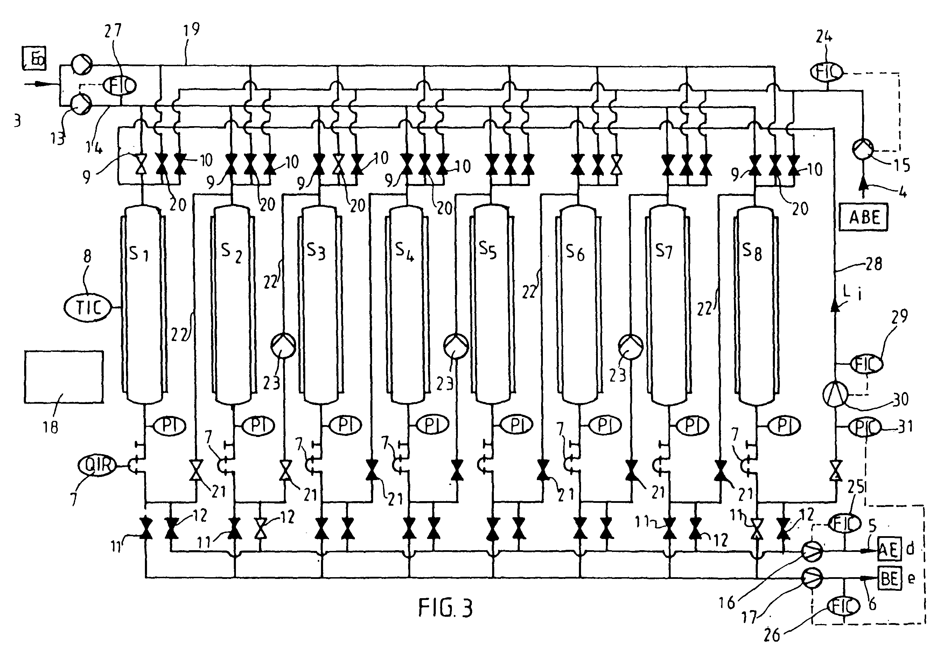

Fig. 3 zeigt ein Fließbild für die technische Realisierung des Verfahrens. Jede der acht hintereinandergeschalteten Säulen $S_1$ bis $S_8$ ist mit einer Flüssigkeitssammel- und Verteilungseinrichtung ausgestattet und enthält das Adsorbens in Form einer kompakten Festbettschüttung mit vorgegebener Höhe, so daß die gewünschte Trennstufenzahl erreicht wird. Die Säulen sind mit Hilfe von Temperaturregelvorrichtungen 8 thermostatisierbar, so daß auch unterschiedliche Temperaturprogramme gefahren werden können. Mit den Solenoidventilen 9 können die Zugabestellen für das Eluens E0 und mit den Solenoidventilen 10 die Zugabestellen für die Zulaufmischung ABE entlang der Säulenschaltung angewählt (geöffnet) werden. Die Entnahmestellen für das Raffinat BE werden mittels der Solenoidventile 11 und für den Extrakt AE mittels der Solenoidventile 12 angewählt. Das Eluens E0 wird durch die Förderpumpe 13 in der Leitung 14 und die Zulaufmischung ABE durch die Förderpumpe 15 in die Anlage eingespeist. Der Extrakt AE wird mit Hilfe der Förderpumpe 16 am Extraktablauf 5 und das Raffinat BE mit Hilfe der Förderpumpe 17 am Raffinatablauf 6 abgezogen. Zu Beginn eines Zyklus werden z.B. die Zugabeventile 9 für das Eluens E0 an der Säule $S_1$ und die Zugabeventile 10 für die Zulaufmischung ABE an der Säule $S_5$, sowie die Entnahmeventile 11 für das Raffinat BE an der Säule $S_6$ und die Entnahmeventile 12 für den Extrakt AE an der Säule $S_2$ geöffnet. Dabei sind alle anderen Ventile der Ventilgruppen 9,10,11 geschlossen. Beim nächsten Takt werden dann jeweils die Ventile der darauffolgenden Säulen betätigt. Nach 8 Takten ist jeweils ein Zyklus abgeschlossen. Die Betätigung der Ventile nach vorgegebenen Taktzeiten erfolgt durch einen Prozeßrechner 18, in dem auch der Prozeßalgorithmus hinterlegt ist. Somit kann durch die Wanderung der Zugabe- und Entnahmestellen auf der Grundlage vorgegebener Taktzeiten in der Säulenschaltung die Bewegung des Festbettes gegenüber dem Eluens simuliert werden.

Über die Leitung 19 und die Ventile 20 können ggf. Spülflüssigkeiten durch die Säulen geleitet werden. Dabei können mit Hilfe der Ventile 21 in den Verbindungsleitungen 22 einzelne Säulen oder Säulengruppen ausgeblendet werden. Im Prozeßbetrieb sind die Ventile 21 stets geschlossen. Zur Reduzierung des Systemdruckverlusts, der durch die Zulaufpumpen 13 und 15 überwunden werden muß, sind in den Verbindungsleitungen 22 pulsationsfreie Boosterpumpen 23 eingebaut.

Der Prozeßrechner kann unterschiedliche Prozeßvarianten einschließlich variabler Taktzeiten, Säulenausblendungen und Spül- und Regenerationszyklen steuern. Damit gelingt auch die Durchführung schwierigster Trennaufgaben mit hohen Taktzeiten bis zu 60 Minuten und mehr auch beim Einsatz von inkompressiblen und quellenden Adsorbentien. Insbesondere ist gewährleistet, daß druckempfindliche chirale Polymeradsorbentien nicht durch Druckwechselbeanspruchung geschädigt werden, so daß auch mit solchen Adsorbentien lange Standzeiten realisiert werden können.

Für den stationären kontinuierlichen Betrieb ist eine sorgfältige Einhaltung der Mengenströme der vier dem Prozeß zugeleiteten und abgeführten Teilströme unabdingbare Voraussetzung. Dies wird durch folgendes Regelungskonzept erreicht:

1. Der Produktstrom ABE, der Extraktstrom AE und der Raffinatstrom BE werden konstant gehalten. Zu diesem Zweck werden die Mengenströme durch Massenstromsensoren überwacht, deren Ausgangssignale PID-Regler (FIC) 24, 25 und 26 ansteuern, die die Drehzahlen der zugeordneten zwangsfördernden Pumpen 15, 16, 17 (z.B. Zahnradpumpen) so nachregeln, daß die Abweichungen der Massenströme von den vorgegebenen Sollwerten minimiert werden. Anstelle der zwangsfördernden Pumpen können natürlich auch andere Pumpen in Verbindung mit geeigneten Regelventilen als Stellglieder zur Mengenstromregelung eingesetzt werden.

2. Der Eluensstrom EO wird in Abhängigkeit des Drucks in der Zulaufleitung 14 mit Hilfe des mit der Förderpumpe 13 verbundenen PID-Reglers 27 derart nachgeregelt, daß bei einem Druckanstieg die Drehzahl der Förderpumpe 13 und damit der Zulaufstrom reduziert und bei einem Druckabfall die Drehzahl entsprechend erhöht und damit der Zulaufstrom vergrößert wird. Dies hat zur Folge, daß sich bei einem konstant vorgegebenen Systemdruck in der Zulaufleitung 14 der Eluensmengenstrom in Abhängigkeit der anderen Flüssigkeitsteilströme ABE, AE und BE frei einstellen kann. Auf diese Weise stellen sich in dem geschlossenen flüssigen und daher inkompressiblen System im kontinuierlichen Betrieb zwangsläufig bilanzgerechte Strömungsverhältnisse ein, ohne daß es zu einer Überschreitung von vorgegebenen Druckgrenzwerten kommt. Der Mengenstrom $L_i$ des internen Kreislaufes, der von der letzten Säule $S_8$ durch die Rückleitung 28 zur ersten Säule $S_1$ strömt, wird mit Hilfe eines Massenstromreglers 29 und der Pumpe 30 konstant gehalten. Aufgrund der Änderung der Massenströme zwischen den wandernden Zugabe- und Entnahmestellen entlang der Säulenschaltung müssen dabei entsprechend den Teilströmen $L_1$ - $L_4$ (s. Fig. 1 und 2) nacheinander die vier unterschiedlichen Sollwerte $L_1$ - $L_4$ für den internen Kreislaufstrom $L_i$ vorgegeben werden. Auf diese Weise wird der inneren Mengenstrombilanz für die Teilströme Rechnung getragen.

Der Sollwert des Druckes in der Eluenszuleitung 14 wird so eingestellt (vorgewählt), daß der Druck an den Ausgängen 5 und 6 für das Extrakt AE und das Raffinat BE stets deutlich über dem Sättigungsdampfdruck des betreffenden Eluens unter Berücksichtigung des Druckverlustes in der Säulenschaltung liegt. In der Regel wird ein Sicherheitsabstand von 50 bis 150 kPa ausreichend sein.

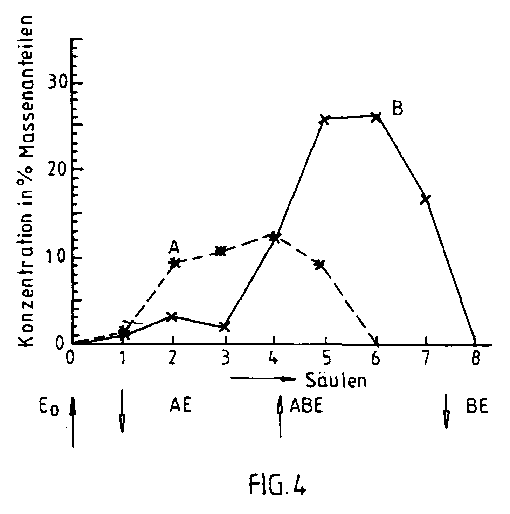

Besonders gute Trennbedingungen erhält man, wenn die Zuspeisung des Zulaufes ABE in Abhängigkeit des sich einstellenden Konzentrationsprofils längs der Säulenschaltung jeweils an der optimalen Stelle erfolgt. Die Bedingung für diese Optimierung besteht darin, daß die aufzutrennende Mischung ABE zwischen den Konzentrationsmaxima der beiden Komponenten oder Fraktionen aufgegeben wird und simultan damit bewegt wird. Das Konzentrationsprofil kann mit Hilfe einer On-line-Analyse durch Untersuchung der an den Meßstellen 7 abgezweigten Proben gewonnen werden. Zu diesem Zweck werden die abgezogenen Proben On-line mit Hilfe spektroskopischer Methoden analysiert. In den meisten Fällen genügt jedoch auch eine Off-line-Analyse, z.B. mittels Gaschromatographie oder HPLC, wenn sich ein stationärer Prozeßzustand herausgebildet hat und die Verschiebung des Konzentrationsprofils als Funktion der Zeit bei vorgegebenen Prozeßparametem einmal empirisch bestimmt wurde und somit eine bekannte Prozeßfunktion darstellt. Die Off-line-Messung entspricht einer Momentaufnahme des Konzentrationsprofils zu einem bestimmten Zeitpunkt. Eine solche Momentaufnahme ist in dem Diagramm gemäß Fig. 4 gezeigt, wobei als Abszisse die Zahl der Säulen und als Ordinate die Konzentration der beiden Komponenten A und B (gestrichelte Kurve A, durchgezogene Kurve B) in %-Massenanteilen aufgetragen ist. Bei dem Versuch gemäß Fig. 4 betrug die gesamte Festbettlänge des Absorbens 8 x 125 mm. Die Proben wurden nach jeder Säule beim ersten Takt nach 67 vollständigen Zyklen an allen 8 Säulen (Meßstellen 7) gleichzeitig entnommen und mittels HPLC (Hochdruck-Flüssigkeitschromatographie) untersucht. Es wurde gefunden, daß der optimale Aufgabeort für die aufzutrennende Mischung ABE zwischen den Konzentrationsmaxima der beiden Komponenten A, B liegt. Zum Zeitpunkt der Momentaufnahme gemäß Fig. 4 liegt dieser Aufgabeort gerade zwischen der vierten und fünften Säule. Die Momentaufnahme des Konzentrationsprofils wird zweckmäßig von Zeit zu Zeit wiederholt, wobei der zeitliche Abstand im stationären Betrieb mehrere vollständige Zyklen (z.B. 2 bis 3) betragen sollte.

Als Eluens sind alle gebräuchlichen Lösungsmittel oder Gemische aus ihnen geeignet, z.B. wie geradkettige, verzweigtkettige Aliphate oder cycloaliphatische Kohlenwasserstoffe, Benzol, alkyl- und halogensubstituierte Benzole, aliphatische Halogenkohlenwasserstoffe, aliphatische und aromatische Alkohole, Ester, Ether, Amine und Ketone. Auch Wasser und Kombinationen aus wassermischbaren Vertretern der angeführten organischen Lösungsmittel können eingesetzt werden.

Die zum Einsatz: kommende chirale stationäre Phase muß im Hinblick auf die spezielle Trennaufgabe ausgesucht werden. Hinsichtlich des Temperaturbereiches gibt es keine besonderen Beschränkungen; vorzugsweise wird der Prozeß zwischen 0°C und 120°C, in speziellen Fällen bis zu 240°C, betrieben, wobei darauf geachtet werden muß, daß der Systemdruck p oberhalb des Dampfdruckes $p_{oi}$ gemäß p> 2...3,5 $p_{oi}$ liegt.

Beispiele

Die chromatographischen Trennungen von racemischen Gemischen im kontinuierlichen Gegenstrombetrieb wurden in einer Laboranlage aus 8 hintereinandergeschalteten Glassäulen DN 50 * 125 mm (Versuche I bis III) bzw. Stahlsäulen DN 20 * 125 mm (Versuch IV) durchgeführt. Die exakte Einhaltung der korrekten Taktzeiten und Bilanzen wurde durch eine rechnergesteuerte Ventilschaltung ermöglicht.

Versuch I:

Getrennt wurde racemisches 2-tert-Butyl-3-(2,4-dichlorbenzoyl)oxazolidin-5-on (A+B). Die Säulen waren hierbei mit der chiralen Trennphase aus vernetztem Poly(L-phenylalanin-d-methylester-acrylamid) als selektivem Adsorbens gefüllt. Als Eluens EO wurde ein Gemisch aus 85/15 Gew.-% Toluol und THF bei einer Temperatur von 50,1°C eingesetzt. Der Zulaufstrom ABE bestand aus 36 g Mischung (A+B+E) mit 3,33 g A+B pro Stunde. Die Taktzeit lag bei 1537 s. Der Kreislaufstrom ($L_o$) betrug 500 g/h, die Eluenszugabe 3 (E) 90 g/h. Es konnte ein Extraktstrom 5 (BE) von 57 g/h und ein Raffinatstrom 6 (AE) von 69 g/h mit einer Konzentration der Enantiomeren ((A+B)/(A+B+E)) im Eluat von 3,7 bis 4,3 % bei einer optischen Reinheit von 90 bis 95 % ee erzielt werden.

Die sorgfältige Einhaltung der Mengenströme der vier dem Prozeß zugeleiteten und abgeführten Ströme wurde hier durch die direkte Regelung des zulaufenden Produktstromes (ABE), des abfließenden Extrakt-(AE) und Raffinatstromes (BE) kontrolliert. Die Messung der Massenströme erfolgte mit einem temperaturkompensierten Corioliskäft-Biegeschwinger. Als Stellgröße für die PID-Regler 24, 25, 26 fungierte die Drehzahl von zwangsfördernden Zahnradpumpen 15, 16, 17. Der Eluensstrom $E_O$ wurde über den Druck in der Zulaufleitung 14 als Regelgröße (Druckregler 27) kontrolliert, so daß sich im geschlossenen flüssigen, inkompressiblen System bilanzgerechte Strömungsverhältnisse einstellen. Als Sollwert für den Druck hat sich ein Wert von 65 kPa zuzüglich des Druckverlustes der Säulen von 8*25 kPa bewährt. Bei Druckanstieg wurde die Drehzahl der Zulaufpumpe 13 und somit der Zulaufstrom reduziert, bei Druckabfall wurde die Drehzahl entsprechend erhöht und somit auch der Zulaufstrom. Der interne Kreislaufstrom $L_i$ in der Rückleitung 28 wurde durch einen Massenstromregler 29 mit einer Pumpe 30 als Stellglied geregelt. Durch die ortsfeste Regelung in den aufeinanderfolgenden Phasen des Prozesses mußte mit vier unterschiedlichen Sollwerten für den Kreislaufstrom $L_i$ gearbeitet werden:

$$L_1 = = 587 \text{ g/h}, L_2 = 530 \text{ g/h}, L_3 = 566 \text{ g/h}, L_4 = 500 \text{ g/h}.$$

Um sicherzustellen, daß die aufzutrennende Mischung zwischen den Konzentrationsmaxima der beiden Komponenten oder Fraktionen aufgegeben wird, wurde das Konzentrationsprofil mit Hilfe einer Off-line-HPLC-Analyse verfolgt. Dazu wurden an den Säulenabläufen mit gasdichten HPLC-Spritzen sehr kleine Proben (ca. 1 µl) während des Betriebes entnommen und sofort analysiert. Die Momentaufnahme des Profils lag nach etwa 90 Minuten vor. Beim Anfahren wurde nach jedem vollen Zyklus, also acht Takten, das Konzentrationsprofil aufgezeichnet. Nach sechs vollen Zyklen im stationären Betrieb erfolgte die Profilaufnahme nur zwei- bis dreimal am Tag. Die Anlage lief rund um die Uhr ohne Störungen.

Versuch II:

Als Eluens wurde ein Lösungsmittelgemisch aus 80/20 Gew.-% Toluol und THF eingesetzt. Bei einer Temperatur von 49,8°C wurde ein Zulaufstrom 4 von 36 g Mischung (A+B+E) mit 5,04 g A+B pro Stunde aufgegeben. Die Taktzeit lag bei 1647 s. Der Kreislaufstrom ($L_o$) betrug 500 g/h, die Eluenszugabe 3 (EO) 87 g/h. Es konnte ein Extraktstrom 5 (BE) von 57 g/h und ein Raffinatstrom 6 (AE) von 66 g/h mit einer Konzentration der Enantiomeren im Eluat von 4,1 bis 4,4 % bei einer optischen Reinheit von 91 bis 97 % ee erzielt werden.

Versuch III:

Im Unterschied zu Versuch II wurde mit variablen Taktzeiten gearbeitet. Die Taktzeiten lagen für die einzelnen Phasen des Prozesses bei 1587, 1647, 1543, 1587, 1647, 1483, 1587 und 1647 s. Der Kreislaufstrom ($L_o$) betrug 500 g/h, die Eluenszugabe 3 (EO) 102 g/h. Es konnte ein Extraktstrom 5 (BE) von 64 g/h und ein Raffinatstrom 6 (AE) von 74 g/h mit einer Konzentration der Enantiomeren im Eluat von 4,3 bis 4,5 % bei einer optischen Reinheit von 94 bis 97,2 % ee erzielt werden.

Versuch IV:

Getrennt wurde das in Versuch I angeführte Racemat an kieselgelgebundenem Poly-L-phenylalaninethylesteracrylamid.

Eingesetzt wurde ein Lösungsmittelgemisch aus 80/20 Gew.-% n-Heptan und THF. Bei einer Temperatur von 30.0°C wurde ein Zulaufstrom 4 von 65 g Mischung (A+B+E) mit 6,5 g A+B pro Stunde aufgegeben. Die Taktzeit lag bei 1560 s. Der Kreislaufstrom ($L_o$ betrug 156 g/h, die Eluenszugabe (EO) 117 g/h. Es konnte ein Extraktstrom 5 (BE) von 100 g/h und ein Raffinatstrom 6 (AE) von 83 g/h mit einer Konzentration der Enantiomeren im Eluat von 1,7 bis 3,3 % bei einer optischen Reinheit von 98 % ee erzielt werden.

**EP 0 586 385 B2**

**Patentansprüche**

1. Verfahren zur Trennung eines chiralen Stoffgemisches mit Hilfe eines Gegenstrom-Chromatographieverfahrens, bei dem einer aus einer Vielzahl von hintereinandergeschalteten, mit einem Adsorbens gefüllten Chromatographiesäulen bestehenden Säulenschaltung (2) das zu trennende Stoffgemisch ABE und das Eluens kontinuierlich zugeführt werden und an anderen Stellen der Säulenschaltung (2) ein die Komponente A enthaltender Extraktstrom AE, sowie ein die Komponente B enthaltender Raffinatstrom BE kontinuierlich entnommen werden und bei dem eine Relativbewegung zwischen einer aus dem Stoffgemisch ABE und dem Eluens EO bestehenden flüssigen, mobilen Phase und dem Adsorbens in fester Phase durch sequentielles Öffnen von Flüssigkeitszugabe und -entnahmestellen (9, 10, 11, 12) entlang der Säulen $S_1$ bis $S_8$ erzeugt wird, **dadurch gekennzeichnet, dass** der mit einem chiralen Polymer als Adsorber gefüllten Säulenschaltung (2) ein enantiomeres Stoffgemisch ABE zugeführt wird, wobei die Mengenströme EO, ABE, AE und BE konstant geregelt werden, während bei einem voreingestellten Systemdruck in der diesen Teilstrom führenden Zuleitung (14) so nachgeregelt wird, dass der Systemdruck konstant bleibt und wobei der in Strömungsrichtung nach dem Stellglied (13) herrschende Systemdruck in der Zuleitung (14) als Regelgröße benutzt wird.

2. Verfahren nach Anspruch 1, **dadurch gekennzeichnet, dass** im stationären Betrieb die Konzentrationsverläufe der beiden Komponenten A, B längs der Säulenschaltung (2) gemessen werden und dass die Einspeisung (4) des zu trennenden Stoffgemisches ABE an der dem Schnittpunkt der beiden Konzentrationsprofile nächst benachbarten Aufgabestellen zwischen den Säulen $S_1$ bis $S_8$ erfolgt.

3. Verfahren nach Anspruch 1, **dadurch gekennzeichnet, dass** als Adsorbens vernetzte Perlpolymerisate verwendet werden, die aus optisch aktiven Acryl-, Methacryl- und $\alpha$-Fluoracrylamiden hergestellt sind.

4. Verfahren nach Anspruch 1, **dadurch gekennzeichnet, dass** als Adsorbens Kieselgele verwendet werden, die mit Polymeren belegt sind, die aus optisch aktiven Acryl-, Methacryl- und $\alpha$-Fluoracrylamiden hergestellt sind.

5. Verfahren nach Anspruch 1, **dadurch gekennzeichnet, dass** die Perlpolymerisate gemäß Anspruch 3 eine Korngröße zwischen 10 und 150 μm und einen Quellungsgrad zwischen 3 und 9 ml/g aufweisen.

**Claims**

1. A process for separating a mixture of chiral substances using a countercurrent chromatography process in which the mixture of substances ABE to be separated and the eluant are continuously fed to a column circuit (2) consisting of a number of chromatography columns filled with an adsorbent material connected in series, and an extract stream AE containing component A and a raffinate stream BE containing component B are continuously removed at another point in column circuit (2), and in which relative movement between a liquid mobile phase consisting of the mixture of substances ABE and the eluant EO and the adsorbent material in the solid phase is produced by sequential opening of the liquid feed and liquid withdrawal points (9, 10, 11, 12) along columns $S_1$ to $S_8$, **characterised in that** an enantiomeric mixture of substances ABE is fed to the column circuit (2) filled with a chiral polymer as adsorber, wherein the flow rates ABE, AE and BE, are kept constant, whereas EO is readjusted to a preset system pressure in the piping carrying this sub-stream (14), in such a way that the system pressure remains constant, and wherein the system pressure prevailing in piping (14) after controller (13), in the direction of flow, is utilised as the controlled quantity.

2. A process according to Claim 1, **characterised in that**, under steady-state operation, the changes in concentration of the two components A and B are measured along the column circuit (2) and the feeding procedure (4) for the mixture of substances ABE being separated takes place between columns $S_1$ and $S_8$ at the next feed point following the cross-over point of the two concentration profiles.

3. A process according to Claim 1, **characterised in that** cross-linked bead polymers which are prepared from optically active acrylamides, methacrylamides and $\alpha$-fluoroacrylamides are used as adsorbent material.

4. A process according to Claim 1, **characterised in that** silica gels which are coated with polymers which are prepared from optically active acrylamides, methacrylamides and $\alpha$-fluoroacrylamides are used as adsorbent material.

5. A process according to Claim 1, **characterised in that** bead polymers in accordance with Claim 3 have a particle size between 10 and 150 µm and a degree of swelling between 3 and 9 ml/g.

**Revendications**

1. Procédé pour séparer un mélange de substances chiral à l'aide d'un procédé de chromatographie à contre-courant dans lequel le mélange de substances à séparer EAB et l'éluant sont amenés en continu à un circuit de colonnes (2) consistant en une multiplicité de colonnes de chromatographie remplies d'adsorbant et branchées les unes à la suite des autres et, à d'autres points du circuit de colonnes (2), un courant d'extrait AE contenant le composant A et un courant de raffinat BE contenant le composant B sont prélevés en continu et dans lequel un mouvement relatif entre une phase mobile liquide consistant en le mélange de substances ABE et en l'éluant EO et l'adsorbant en phase fixe est engendré par ouverture séquentielle de points d'apport et de soutirage de liquide (9, 10, 11, 12) le long des colonnes $S_1$ à $S_8$, **caractérisé en ce qu'**un mélange de substances énantiomérique ABE est amené au circuit de colonnes (2) rempli d'un polymère chiral comme adsorbant, les courants massiques ABE, AE et BE étant régulés de manière à être constants, tandis que EO est post-régulé pour une pression du système préétablie dans la conduite d'alimentation (14) transportant ce courant partiel de telle manière que la pression du système reste constante et la pression du système régnant dans le sens de l'écoulement en aval de l'organe de réglage (13) étant utilisée comme grandeur de régulation dans la conduite d'amenée (14).

2. Procédé selon la revendication 1, **caractérisé en ce que**, en fonctionnement stationnaire, les évolutions de concentration des deux constituants A, B sont mesurées le long du circuit de colonnes (2) et **en ce que** l'introduction (4) du mélange de substances à séparer ABE a lieu aux points d'alimentation les plus proches du point d'intersection des deux profils de concentration entre les colonnes $S_1$ à $S_8$.

3. Procédé selon la revendication 1, **caractérisé en ce que** des produits de polymérisation en perles réticulés, qui sont préparés à partir d'acryl-, méthacryl- et $\alpha$-fluoroacrylamides optiquement actifs, sont utilisés comme adsorbant.

4. Procédé selon la revendication 1, **caractérisé en ce que** des gels de silice qui sont chargés avec des polymères qui sont préparés à partir d'acryl-, méthacryl- et $\alpha$-fluoroacrylamides optiquement actifs sont utilisés comme adsorbant.

5. Procédé selon la revendication 1, **caractérisé en ce que** les produits de polymérisation en perles selon la revendication 3 présentent une taille de grains comprise entre 10 et 150 µm et un degré de gonflement compris entre 3 et 9 ml/g.

FIG.1

FIG.2

FIG. 3

EP 0 586 385 B2

10

FIG.4